(19)

Europäisches Patentamt
European Patent Office
Office européen des brevets

(11) **EP 4 364 575 A1**

(12) **EUROPEAN PATENT APPLICATION**

(43) Date of publication:
**08.05.2024 Bulletin 2024/19**

(21) Application number: **23207797.4**

(22) Date of filing: **03.11.2023**

(51) International Patent Classification (IPC):
*A23B 7/152* (2006.01)  *G01N 33/00* (2006.01)
*G01N 33/02* (2006.01)  *G06Q 10/08* (2024.01)
*G06Q 30/02* (2023.01)  *G06Q 50/02* (2024.01)

(52) Cooperative Patent Classification (CPC):
**G06Q 10/0832; A23B 7/152; G01N 33/0036;
G01N 33/025; G06Q 30/0278; G06Q 50/02**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **03.11.2022 NL 2033458**

(71) Applicant: **Nature's Pride
2676 LV Maasdijk (NL)**

(72) Inventor: **WIND, Stephanus Leendert Jan
2565 MB DEN HAAG (NL)**

(74) Representative: **Arnold & Siedsma
Bezuidenhoutseweg 57
2594 AC The Hague (NL)**

(54) **METHOD, DEVICE AND INSTALLATION FOR RIPENING CLIMACTERIC FRUITS**

(57)    The invention relates to a computer implemented method for ripening climacteric fruits in an enclosure comprising the steps of:

a. setting a target for a degree of ripening of the fruits in the enclosure;

b. at least periodically analyzing a composition of the atmosphere in the enclosure;

c. deriving, by a processor, based on a plant physiological model describing the ripening process in the fruits, an instantaneous degree of ripening of the fruits from the analyzed composition;

d. predicting, by the processor, based on the model and the derived degree of ripening, a moment in time when the target will be met; and

e. signaling, by the processor, the predicted moment.

The invention also relates to a device for ripening climacteric fruits in an enclosure comprising:

A. a first setting module for setting a target for a degree of ripening of the fruits in the enclosure;

B. a control module connected to the first setting module and including a plant physiological model describing the ripening process and a processor;

C. an analyzer module connected to the control module for at least periodically analyzing a composition of the atmosphere in the enclosure;

wherein the processor is arranged for:

deriving, based on the model, an instantaneous degree of ripening of the fruits from the analyzed composition of the atmosphere;

predicting, based on the model and the derived degree of ripening, a moment in time when the target will be met; and

signaling the predicted moment.

FIG. 8

EP 4 364 575 A1

**Description**

**Technical Field**

The invention generally relates to providing optimal ripening conditions for climacteric fruits, and more in particular to a method, a device and an installation for ripening climacteric fruits.

**Background**

Climacteric fruits are fruits which can ripen after having been harvested, like e.g. bananas or avocados. It is important to control ripening conditions such that the fruits will be at an optimal stage of ripening when they reach an end user or consumer. This optimal stage of ripening is indicated as "ready to eat". When climacteric fruits are not yet ripened to the "ready to eat" state, the consumer cannot eat the fruits at an intended moment, e.g. directly after purchasing the fruits. On the other hand, if the climacteric fruits reach the "ready to eat" state before reaching the consumer, they may be overripe at the intended moment of consumption. Since ripening of the climacteric fruits is a constant process from the moment of harvest to consumption, which may be accelerated or decelerated as a result of ambient conditions, it is hard to regulate ripening such as to ensure that the fruits will reach the consumer in the "ready to eat" state.

Typically, climacteric fruits are harvested when they are mature but not yet ripe, and then transported to ripening facilities, which may be far removed from the location where the fruits are grown. During transport to the ripening facilities, storage at these facilities and subsequent transport from the ripening facilities to the consumers, climacteric fruits will undergo postharvest ripening. The rate at which the fruits ripen depends i.a. on temperature during transport and storage and on the composition of the atmosphere in which the fruits are transported and stored.

Conventionally, climacteric fruits are transported under such conditions that they are still unripe when they reach a ripening facility. There the fruits are ripened to a desired degree under controlled conditions in a ripening room. The ripening process is overseen by specialist operators who monitor the fruits and control the conditions in the ripening room to achieve a desired rate of ripening. Monitoring the fruits includes monitoring their firmness. The operator controls the conditions in the ripening room based on his or her experience.

From the ripening facilities the fruits are transported to points of sale, e.g. wholesalers or supermarkets. During this transport the fruits may ripen further, depending on temperature and composition of the atmosphere.

EP 3 705 888 A2 discloses a system and method for managing ripening conditions of climacteric fruits. The method includes obtaining levels of environment condition parameters (e.g. $CO_2$ emitted, $O_2$ consumed, ethylene emitted, temperature and relative humidity) associated with ripening of the climacteric fruit over time at periodic intervals by using an enclosure enclosing the climacteric fruit. The method further includes performing RGB image analysis on different pictorially reported ripening stages of the fruits. A respiration rate of the climacteric fruit is computed based at least on the levels of the environment condition parameters using Michaelis Menten kinetics model. A level of ethylene is monitored to determine a climacteric peak of ethylene for the climacteric fruit. The climacteric peak is indicative of complete natural ripening of the climacteric fruit. A pre-trained artificial neural network (ANN) model predicts optimal ripening condition of the climacteric fruit based on the respiration rate of the climacteric fruit and the climacteric peak of ethylene.

However, the output of an artificial neural network cannot be readily understood and is not necessarily stable over time, as self-learning may move the output away from the original relationships between the input parameters. Moreover, the system is complex and requires substantial investment in sensors and high-resolution cameras.

Consequently, there is a need for an improved method, device and installation for ripening climacteric fruits.

**Summary**

In accordance with an aspect of the invention, a computer implemented method for ripening climacteric fruits in an enclosure comprises the steps of:

a. setting a target for a degree of ripening of the fruits in the enclosure;
b. at least periodically analyzing a composition of the atmosphere in the enclosure;
c. deriving, by a processor, based on a plant physiological model describing the ripening process in the fruits, an instantaneous degree of ripening of the fruits from the analyzed composition;
d. predicting, by the processor, based on the model and the derived degree of ripening, a moment in time when the target will be met; and
e. signaling, by the processor, the predicted moment,

wherein the plant physiological model is based on a biosynthetic pathway of ethylene in the fruit and on dilution and diffusion of ethylene through skins of the fruits.

In this way the ripening process of the fruits can be automatically monitored and the moment of reaching the desired degree of ripening can be established without any interaction with the fruits or interference with the ripening process. The target may be any degree of ripening which will allow the fruits to be delivered to a customer in a state specified by that customer. The use of a predictive model renders the process objective and independent of the experience of an operator. The prediction of the progress of the ripening process can be based on analysis of the composition of the atmosphere in the enclosure, e.g. by measuring the amount of oxygen, carbon dioxide and/or ethylene in the enclosure. In particular the amount of ethylene in the atmosphere is a strong indicator, from which the state of the ripening process may be derived through the plant physiological model. The method does not require any images to be analyzed, and does not make use of any artificial neural network..

Based on the processor signaling the predicted moment in time, the fruits may be taken from the enclosure for distribution and transport to consumers. Alternatively, the enclosure may be a transport container or a hold of a ship, in which the fruits may be pre-ripened to a first degree of ripening during transport from the grower to the ripening facility. In that case, after being taken from the transport enclosure, the fruits may be introduced into another enclosure, e.g. a ripening chamber, to ripen further to a higher degree of ripening.

In an embodiment of the method, the model may describe the ripening process based on production of ethylene in the fruits, and the moment in time when the target will be met may be predicted after the production of ethylene in the fruits has reached a peak. This peak in ethylene production, which can be refereed to as a "climacteric peak", can be observed clearly and forms a distinctive identifier for monitoring progress of the ripening process.

In a further embodiment of the method, the model may describe the ripening process as a function of a weighted combination of temperature and time. It has been found that the temperature in the enclosure is an important factor in controlling the rate of ripening of the fruits. By using a weighted combination of temperature of the atmosphere in the enclosure and the time the fruits spend in the enclosure, the model can predict when the target for the degree of ripening may be expected to be met. The weighted combination may take into account that the ripening process is slowed down considerably when the temperature is lowered. The reduction of the rate of ripening may not be a linear function of the lower temperatures; in fact, when the temperature falls below a certain threshold the ripening process almost comes to a standstill. This relationship may be reflected by assigning a much smaller weight to storage time at temperatures below the threshold than to storage time above the threshold.

For instance, the weighted combination may be defined as a "temperature-age", which may be expressed in temperature-hours (TH), i.e. a period of time multiplied by an average temperature during that period. One hour at a temperature of e.g. 21°C could lead to a temperature-age of 21 TH. To reflect the much slower rate of ripening at lower temperatures, a weighing factor of e.g. 0.1 may be applied when the average temperature is below a threshold of e.g. 14°C. In that case one hour of storage at a temperature of e.g. 12°C would lead to a temperature-age of 1.2 TH.

In an embodiment of the method, the model may define a relationship between the ethylene production in the fruits and the amount of ethylene in the atmosphere in the enclosure. Since it takes time for the ethylene produced in the fruits to be exhausted into the enclosure, the measured amount of ethylene does not accurately reflect the actual degree of ripening of the fruits. The model may compensate for the time lag between production and measurement of ethylene.

In another embodiment of the method, the target may be associated with a peak in the amount of ethylene in the atmosphere. Such a peak may be an indication that the fruits are at an optimum degree of ripening.

In yet another embodiment of the method, the model may define this atmospheric peak as point of substantially complete ripening of the fruits when it is reached within a predetermined range of temperature and time. It has been found that multiple peaks may be observed over time, including an early peak when the fruit is still unripe and a late peak when the fruit is overripe. By defining a window in a temperature and time space, the method may ensure that only the true optimum in ripeness is identified by the atmospheric peak.

In an embodiment of the method, the target to be set may be determined based on a desired degree of ripening of the fruits at an consumer and an expected ripening during transport from the enclosure to the consumer. In this way the ripening in the enclosure may be stopped before the fruits reach an optimum degree of ripening, to prevent them from becoming overripe during transport to the consumer.

In a further embodiment of the method, based on the model the processor may determine the target to be set based on an expected time of transport from the enclosure to the consumer and an expected temperature during transport. Use of the model may thus be extended to the transport phase after leaving the controlled atmosphere of the enclosure.

In another embodiment, the target to be set may be determined such that the fruit is at a customer specified degree of ripening when reaching the consumer. Since the trend in consumption is shifting towards instant satisfaction, the fruit is usually required to be "ready to eat" when it reaches the consumer. However, other degrees of ripening, like e.g. "triggered" may be specified by customers.

In an embodiment, the method may further comprise the steps of:

f. setting, within a predetermined bandwidth, a moment in time when the target must be met;

g. comparing, by the processor, the set moment with the predicted moment;

h. if the predicted moment differs from the set moment, determining, by the processor, based on the model, a value of at least one atmospheric parameter in the enclosure that will allow the predicted moment to match the set moment; and

i. controlling, by the processor, an atmospheric control system connected to the enclosure to set the determined value of the at least one atmospheric parameter.

In this way the ripening process of the fruits can be automatically controlled so as to bring the fruits exactly to a desired degree of ripening at a desired point of time. Control of the ripening process may require a relatively small number of control actions to set and maintain values of atmospheric parameters like e.g. the temperature in the enclosure. Implementing the method requires a relatively limited investment when compared with prior art methods.

In an embodiment, the method may further comprise the steps of:

j. setting, based on a measured firmness of the fruits prior to ripening in the enclosure, an initial value for a degree of ripening of the fruits; and

k. deriving, by the processor, based on an alternative plant physiological model describing the ripening process in the fruits, a progress of the ripening from the periodically analyzed composition of the atmosphere in the enclosure.

In this way the alternative model may be used to provide an indication of the momentary state of the ripening process. Firmness is an indication of the degree of ripening which may be measured fairly easily in a small sample taken from a batch of fruits. And when using proper instruments, a firmness measurement may be non-destructive, so that it does not affect the value of the batch of fruits. The combined use of two different plant physiological models provides optimum control over the ripening process of the climacteric fruits.

In yet another embodiment, multiple batches of fruits may be ripened in a single enclosure, and the batches may be selected and positioned in the enclosure based on corresponding initial values. In this way batches of fruits having a similar degree of ripeness may be jointly brought to the target degree of ripening by being stored in the same enclosure for the same amount of time. This leads to an optimum utilization of the available capacity in the enclosures and prevents the enclosures from having to be opened frequently to (partly) load or unload batches due to variation in ripening.

In accordance with another aspect of the invention, a device for ripening climacteric fruits in an enclosure is provided. This device comprises:

A. a first setting module for setting a target for a degree of ripening of the fruits in the enclosure;

B. a control module connected to the first setting module and including a plant physiological model describing the ripening process and a processor;

C. an analyzer module connected to the control module for at least periodically analyzing a composition of the atmosphere in the enclosure;

wherein the processor is arranged for:

deriving, based on the model, an instantaneous degree of ripening of the fruits from the analyzed composition of the atmosphere;

predicting, based on the model and the derived degree of ripening, a moment in time when the target will be met; and

signaling the predicted moment, and

wherein the plant physiological model is based on a biosynthetic pathway of ethylene in the fruit and on dilution and diffusion of ethylene through skins of the fruits.

In this way an effective device for automatically monitoring the ripening process of the fruits is provided. The device does not include any cameras, nor any artificial neural network. There is no need for an operator to enter the enclosure to check on the progress of the ripening process.

In an embodiment, the model may be configured to describe the ripening process based on production of ethylene in the fruits, and the processor may be arranged for predicting the moment in time when the target will be met after the production of ethylene in the fruits has reached a peak. As explained above, this so-called "climacteric peak" is a distinctive and easily recognizable point in the ripening process.

In a further embodiment, the model may be configured to describe the ripening process as a function of a weighted combination of temperature and time. As discussed above, weighting the combination of temperature and time may accommodate for the fact that there is a non-linear relationship between the temperature and the rate at which the fruits ripen.

In another embodiment of the device, the model may be configured to define a relationship between the ethylene production in the fruits and the amount of ethylene in the enclosure. This relationship takes account of the time elapsing between the generation of ethylene in the fruits and the diffusion of the ethylene through the fruit skins into the surrounding atmosphere.

In yet another embodiment of the device, the target may be associated with a peak in the amount of ethylene in the atmosphere, which may indicate that the fruits have reached an optimum degree of ripeness.

In a further embodiment of the device, the model may be configured to define this atmospheric peak as point of ripening of the fruits when it is reached within a predetermined range of temperature and time. Depending on conditions during transport and storage, ethylene levels may peak at various moments, and not every peak is indicative of optimum ripening. Defining a window in a range of temperatures and times allows the device to identify only the true optimum in ripeness by the atmospheric peak.

In an embodiment of the device, the control module and the first setting module may be configured to determine the target based on a desired degree of ripening of the fruits at an consumer and an expected ripening during transport from the enclosure to the consumer. Thus, the target may be set at a point where the fruits have not yet reached an optimum degree of ripeness, to avoid the risk of overripening before they reach the consumer.

In a further embodiment of the device, the control module and the first setting module may be configured to determine the target based on an expected time of transport from the enclosure to the consumer and an expected temperature during this transport. In this way the control module may be used to predict further ripening during the transport phase.

In an embodiment, the device further comprises:

D. a second setting module connected to the control module for setting, within a predetermined bandwidth, a moment in time when the target must be met;

wherein the processor is further arranged for:

comparing the set moment with the predicted moment,

if the predicted moment differs from the set moment, determining, based on the model, a value of at least one atmospheric parameter in the enclosure that will allow the predicted moment to match the set moment; and

controlling an atmospheric control system connected to the enclosure to set the determined value of the at least one atmospheric parameter.

In this way an effective device for automatically controlling the ripening process of the fruits is provided.

In yet another embodiment of the device, the model may include at least a prediction algorithm for predicting or calculating, based on the analyzed composition of the atmosphere, a future degree of ripening, and a control algorithm for calculating a setting of the at least one parameter defining the atmosphere in the enclosure. The control algorithm may use the output of the prediction algorithm and calculate optimum conditions for the atmosphere in the enclosure based on the initial setting(s). The prediction algorithm may anticipate changes in the atmosphere directed by the control algorithm.

In an embodiment of the device, the model may further comprise a balancing algorithm for incrementally adjusting settings of the control algorithm until the future degree of ripening as predicted by the prediction algorithm substantially corresponds to the target. The balancing algorithm allows the prediction and control algorithms to cooperate and use each other's outputs.

In a further embodiment of the device, the first and/or second setting module(s) may form part of a user interface. This allows a user to input desired settings into the device.

In another embodiment of the device, the analyzer module may comprise at least one sensor connected with or arranged in the enclosure. The sensor(s) may act periodically or continuously to generate signals which are representative of the amount of one or more components in the atmosphere in the enclosure, which allows variations in the composition of the atmosphere to be detected very quickly.

In an embodiment, the device may further comprise a third setting module connected to the control module for setting an initial value for a degree of ripening of the fruits in the enclosure, wherein the control module and the third setting module may be configured to determine the initial value based on a measured firmness of the fruits prior to ripening in the enclosure, and wherein the control module further comprises an alternative plant physiological model describing the ripening process in the fruits, and is arranged for deriving a progress of the ripening from the periodically analyzed composition of the atmosphere in the enclosure. Entering the initial degree of ripening into the control module provides a good starting point for monitoring the state of ripening during the entire process. The firmness of a sample taken from a batch of fruits may be measured by a non-destructive firmness measuring instrument, and the results of this measurement may be processed to derive a degree of ripening which corresponds with this firmness. The firmness measuring instrument or firmness tester may be part of or connected to the first setting module and/or the control module.

In a further embodiment, the third setting module may form part of the user interface. This allows a user to input all desired settings into the device using a single interface.

In yet another embodiment, which is intended for ripening multiple batches of fruits in a single enclosure, the device may further comprise a module for selecting and positioning the batches to be placed in the enclosure based on corresponding initial values. This selection module may be configured for reading initial values for a plurality of batches, comparing these initial values with each other and selecting pairs or groups of initial values which correspond sufficiently closely. The selection module may further be arranged to provide an output signaling which batches of fruits have sufficiently closely corresponding initial values of the degree of ripening to be further ripened together in a single enclosure.

In accordance with another aspect of the invention, an installation for ripening climacteric fruits may comprise an enclosure, an atmospheric control system connected to the enclosure and a ripening device as described above connected to the atmospheric control system.

The invention will now be illustrated by way of an exemplary embodiment, with reference being made to the annexed drawings, in which:

Fig. 1 is a schematic representation of the various phases of transport and storage of climacteric fruits between harvesting and consumption;
Fig. 2 is a schematic graph illustrating the natural ripening process of climacteric fruits after harvesting;
Fig. 3 is a graph corresponding with Fig. 2 and illustrating a controlled ripening process of climacteric fruits between harvesting and consumption;
Fig. 4 is a flow chart illustrating the various steps of the inventive method;
Fig. 5 is a flow chart illustrating further steps of an embodiment of the method;
Fig. 6 is a schematic representation of the Yang cycle explaining the formation of ethylene in fruits;
Fig. 7 is a schematic graph illustrating exemplary measurements of ethylene in an enclosure as a function of 'temperature age' and showing various peaks;
Fig. 8 is a graph illustrating both measured and calculated levels of ethylene production in an enclosure as a function of 'temperature age' and showing both a measured and a predicted peak;
Fig. 9 is a graph illustrating both measured and calculated levels of firmness of fruits during ripening as a function of 'temperature age'
Fig. 10 is a schematic representation of a ripening installation comprising an enclosure, an atmospheric control system and a ripening device;
Fig. 11 shows a ripening installation including a plurality of enclosures;
Fig. 12 is a schematic illustration of a ripening device in accordance with an embodiment of the invention; and
Fig. 13 is a more detailed illustration of the various elements of the ripening device.

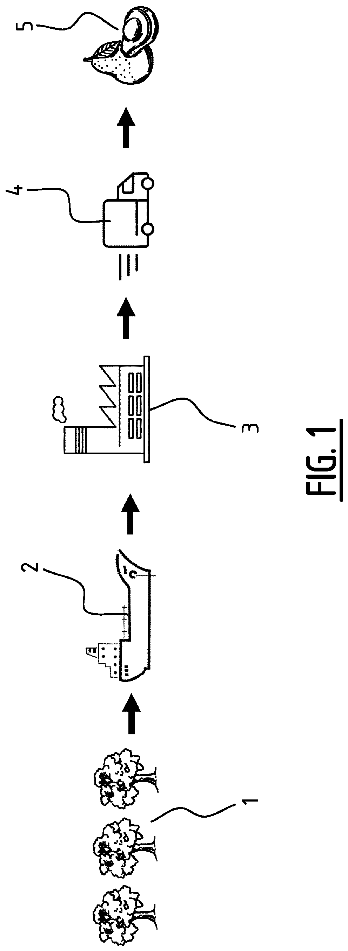

Many fruits are typically grown in warmer regions of the world, like subtropical or tropical regions, and must be transported over relatively large distances to end users or consumers, a large percentage of whom live in Europe or North America. After harvesting at a grower's facility 1 (Fig. 1), fruits will typically be transported in large shipments 2 to an importer or distributor 3 located in the geographical area of the consumers, where they may be temporarily stored. Importers or distributors may have ripening facilities where the fruits are ripened under controlled conditions. From there the fruits will typically be distributed in smaller batches 4, e.g. by road transport to retail outlets 5 close to the consumers.

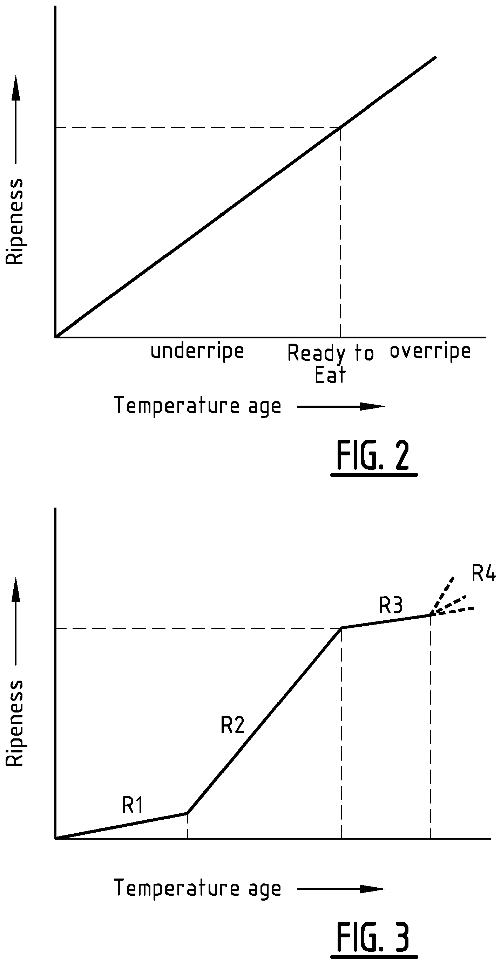

A characteristic of climacteric fruits is that their ripening process continues after harvesting. At some point in time the fruits will reach their optimal state of ripening, also known as "ready to eat" 6 (Fig. 2). Ideally, that is the moment when they reach the consumer. If the consumer is presented with the fruits before they have reached the "ready to eat" state, the fruits will be unripe, i.e. they may still be too hard for consumption, or will not have their full taste. The consumer may decide to defer consumption until the fruits have fully ripened, but in the uncontrolled atmosphere of a home it is hard to predict when this may occur. Conversely, if the fruits reach the consumer some time after having reached the "ready to eat" state, they may be overripe as witnessed e.g. by soft spots or discolorations, and may have lost their full taste.

In actual practice, ripening is not a linear process, but may be accelerated or decelerated, depending on atmospheric conditions. This is schematically illustrated in Fig. 3, where it is shown that during transport from the grower 1 to the importer or distributor 3 ripening is limited (segment R1 of graph). This is due to the fact that the fruits are transported in e.g. refrigerated ships or trucks, so that they are kept at a relatively low temperature for the duration of transport. At the importer or distributor 3 the fruits will be ripened under controlled atmosphere in a ripening facility. Temperatures in the ripening facility will be substantially higher than during transport from the grower, so that the ripening process is accelerated (segment R2). Subsequent transport from the ripening facility to the retail outlet 5 will usually also take place in refrigerated trucks, so that the ripening process is decelerated again (segment R3). Once the fruits reach the store they will normally be displayed in open displays, where the atmosphere is not controlled and temperatures will again be substantially higher than during transport, so that ripening is accelerated (segment R4). The rate of ripening will vary from store to store, as illustrated by the various dotted lines Since the fruits will be handled by different parties during these various stages of transport R1-R4, and since it takes time for the core temperature of the fruits to adapt to the ambient temperature, it is hard to predict the state of ripening that the fruits will have achieved when they reach the consumer.

The invention has for its object to improve uniformity of the ripening process at least when the fruits are stored in the ripening facility, such that their state of ripening when reaching the consumer may be predicted within a certain bandwidth. To this end the invention proposes to monitor the composition of the atmosphere in an enclosure where the fruits are stored and to use this composition in a model to predict a future state of ripening. In a further embodiment, the invention proposes to control at least one atmospheric parameter in the enclosure in order to influence, i.e. speed up or slow down, the ripening process. The composition of the atmosphere is influenced by the ripening process of the fruits, which leads to the production of ethylene in the fruits. This internally produced ethylene is eventually passed from the fruits into the surrounding atmosphere, where it may lead to detectable changes in the composition of the atmosphere.

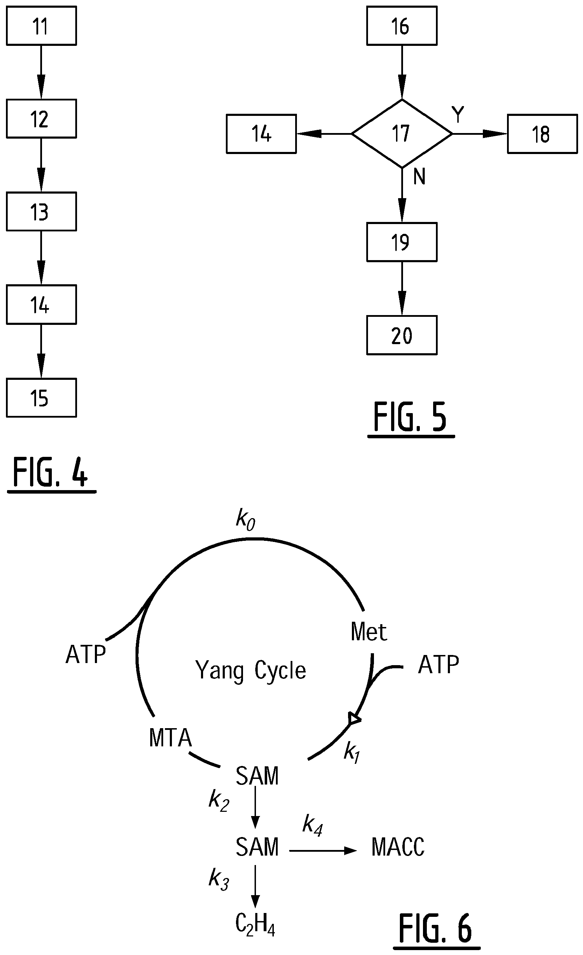

In a computer implemented method 10 for ripening climacteric fruits in an enclosure according to the invention, a target for a degree of ripening of the fruits in the enclosure may be set (Fig. 4, step 11). A composition of the atmosphere in the enclosure may be analyzed at least periodically (step 12), and the results of that analysis may be used by the processor to derive an instantaneous degree of ripening of the fruits in the enclosure (step 13). This derivation may be based on a plant physiological model describing the ripening process in the fruits. Based both on the model and on the degree of ripening as derived in the previous step, a moment in time when the target will be met may be accurately predicted (step 14). This predicted moment may then be signaled (step 15), so that an operator knows when to remove the fruits from the enclosure.

In an embodiment of the invention, the method 10 may further comprise the step of setting a moment in time when the target must be met (Fig. 5, step 16). This moment is set within a predetermined bandwidth, since there is a limit to the temperature to which fruits can be exposed, so that they cannot be ripened instantaneously; ripening takes a certain minimum of time. Then the processor may compare the moment as set in step 16 with the moment as predicted in step 14. This is done in step 17. If the predicted moment corresponds with the set moment, there is no need to take any further action, and ripening may continue as planned (step 18). However, if the predicted moment differs from the set moment, the plant physiological model may be used to determine a value of the at least one atmospheric parameter that will allow the predicted moment to match the set moment (step 19). This determination may again be done by the processor. The processor may then control an atmospheric control system that is connected to the enclosure so as to set the determined value of the atmospheric parameter(s) in the enclosure (step 20).

The plant physiological model which is used to derive the initial degree of ripening at a given moment in time and to predict when the targeted degree of ripening will be achieved is based on emission of ethylene by the fruits during ripening. In particular, the model is based on the biosynthetic pathway of ethylene, including the so-called Yang cycle and the SAM-to-ethylene pathway as shown in Fig. 6 and on the dilution and diffusion of ethylene through the skin of the fruits. The Yang cycle serves to continuously regenerate SAM (S-adenosylmethionine) pools in the fruit tissue to sustain normal rates of ACC (1-amino-cyclopropane-1-carbolxylic acid) production. As the Yang cycle is supplied by ATP (adenosine triphosphate), ATP production through respiration is an important aspect of the model. The SAM-to-ethylene pathway is regulated by ACCs and ACCo (ACC oxidase), which are the two key enzymes considered in the model. The focus is on autocatalytic ethylene production, resulting in up-regulation of these two enzymes by ethylene. The conjugation of ACC with malonate to form MACC (malonyl-ACC) is taken into account in the model as a way to reduce the availability of ACC for ethylene production. In view of the seasonal increase of fruit volume, the dilution of biochemical compounds used in the model is taken into account. Also, diffusion of ethylene across the skin was considered.

Metabolic and biophysical processes are connected in the model. For example, the diffusion of ethylene in the ambient atmosphere depends on the internal ethylene concentration, which in turn depends on the intensity of diffusion and on other processes like e.g. dilution and metabolism. The basic processes described in the model are the Yang cycle, respiration, production of MACC, regulation of ACCs and ACCo, dilution and ethylene release in the ambient atmosphere. Each fruit is described as a compartment separated from an exterior compartment, i.e. the ambient atmosphere, by its skin. Gas exchange occurs by diffusion through the fruit skin. The temperature of the fruit is assumed to be equal to that of the ambient atmosphere.

Fruit growth is considered to influence ethylene production because it is related to respiration, dilution, and to the skin area through which ethylene is released. The temperature is an important external factor controlling respiration in the model. The model also takes into account the effect of internal concentrations of oxygen and carbon dioxide on ethylene through their action on ACCo, which requires oxygen and is inhibited by high concentrations of carbon dioxide.

The main state variables of the system are MACC, ACC, carbon dioxide, oxygen, and ethylene concentrations in the fruit. Information coming from the external compartment, e.g. on an hourly basis, is the temperature and concentrations of oxygen and carbon dioxide in the ambient atmosphere. This information, together with the fruit growth components like dry and fresh mass and dry growth rate, represent the inputs for the model. Using these inputs in combination with a theoretical analysis quantified by the governing equations, the main reaction rates of the ethylene biosynthesis pathway and gas transfer processes may be computed.

In the model, the variation of the MACC concentration is:

$$\frac{d[\text{MACC}]}{dt} = k_4[\text{ACC}] - \frac{[\text{MACC}]}{V}\frac{dV}{dt} \tag{1}$$

wherein $V$ (m³) denotes the volume of the fruits and $k_4$ is a constant.

The rate of variation of ACC concentration can be expressed as:

$$\frac{d[\text{ACC}]}{dt} = \left(k_s\sqrt{\frac{[\text{C}_2\text{H}_4]}{[\text{C}_2\text{H}_{4\text{ref}}]}}[\text{ATP}] - k_o\frac{[\text{O}_2]}{(K_{\text{O}_2}+[\text{O}_2])\left(1+\frac{[\text{CO}_2]}{K_{\text{CO}_2}}\right)}\right.$$
$$\left.\times\sqrt{\frac{[\text{C}_2\text{H}_4]}{[\text{C}_2\text{H}_{4\text{ref}}]}}[\text{ACC}]\right) - k_4[\text{ACC}] - \frac{[\text{ACC}]}{V}\frac{dV}{dt} \tag{2}$$

Fruit respiration in terms of $CO_2$ production can be calculated as:

$$re_{\text{CO}_2} = q_g\frac{dM_{\text{dry}}}{dt} + q_m M_{\text{dry}} Q_{10}^{\frac{(T-20)}{10}} \tag{3}$$

where $M_{dry}$ (g) is the fruit dry mass, $q_g$ (mol/g) the growth respiration coefficient, $q_m$ (mol/gh) the maintenance respiration coefficient at 20°C, $Q_{10}$ the temperature ratio of maintenance respiration, and $T$ (°C) the temperature.

ATP, CO2, and O2 concentrations may then be calculated as a function of fruit dry mass, volume, area, and temperature as follows:

$$[\text{ATP}] = \frac{5\left(q_g\frac{dM_{\text{dry}}}{dt} + q_m M_{\text{dry}} Q_{10}^{\frac{(T-20)}{10}}\right)}{\lambda V + \frac{dV}{dt}} \tag{4}$$

$$[\text{CO}_2] = \left[\text{CO}_2^{\text{air}}\right] + \frac{R_{\text{CO}_2}\left(q_g\frac{dM_{\text{dry}}}{dt} + q_m M_{\text{dry}} Q_{10}^{\frac{(T-20)}{10}}\right)}{A} \tag{5}$$

$$[\text{O}_2] = \left[\text{O}_2^{\text{air}}\right] - \frac{R_{\text{O}_2}\left(q_g\frac{dM_{\text{dry}}}{dt} + q_m M_{\text{dry}} Q_{10}^{\frac{(T-20)}{10}}\right)}{ARQ} \tag{6}$$

while finally the rate of variation of ethylene concentration may be obtained from:

$$\frac{d[\text{C}_2\text{H}_4]}{dt} = k_o\frac{[\text{O}_2]}{(K_{\text{O}_2}+[\text{O}_2])\left(1+\frac{[\text{CO}_2]}{K_{\text{CO}_2}}\right)}\sqrt{\frac{[\text{C}_2\text{H}_4]}{[\text{C}_2\text{H}_{4\text{ref}}]}}[\text{ACC}]$$
$$- \frac{P_{\text{C}_2\text{H}_4}A[\text{C}_2\text{H}_4]}{V} - \frac{[\text{C}_2\text{H}_4]}{V}\frac{dV}{dt}. \tag{7}$$

where the levels of ethylene are a sum of the hourly values from formula (7):

$$[\text{C}_2\text{H}_4] = \Sigma(d[\text{C}_2\text{H}_4]/dt) \tag{8}$$

On the basis of the above-discussed model, and given initial values of fruit dry mass, fruit fresh mass, oxygen and carbon dioxide concentrations to be input to the model, the ethylene production in the fruits may be plotted as a function of temperature and time. In particular, a weighted combination of temperature and time may be used, which may be defined as 'temperature age' (TA) and may be expressed in 'temperature hours' (TH). In a practical embodiment, temperature age may simply be defined as the time elapsed from the start of the ripening process multiplied by the (average) temperature during that time:

$$ TA = \Sigma \text{ (time * temperature)} \tag{9} $$

with time being expressed - in this embodiment - in hours and temperature (averaged over each one-hour interval) being expressed in degrees Celsius. In order to compensate for the fact that ripening slows down dramatically when the temperature falls below a certain threshold, a weighing factor may be employed. In this embodiment the weighing factor is set at 0.1 for temperatures below 14°C:

$$ TA \text{ (low temperature)} = 0.1 * \Sigma \text{ (time * temperature)} \tag{10} $$

It is clear that other units of time may be used, e.g. intervals of 15 minutes or intervals of 3 hours, if a more or less accurate representation is required. And instead of a single temperature threshold defining two weighing factors (1 and 0.1), more thresholds and weighing factors may be defined to create a more accurate representation of the temperature dependency of the ripening process. In fact, the weighing factor could be derived from a look-up table presenting a large number of temperature and associated weighting factors, or it could even be expressed as a continuous function of the temperature.

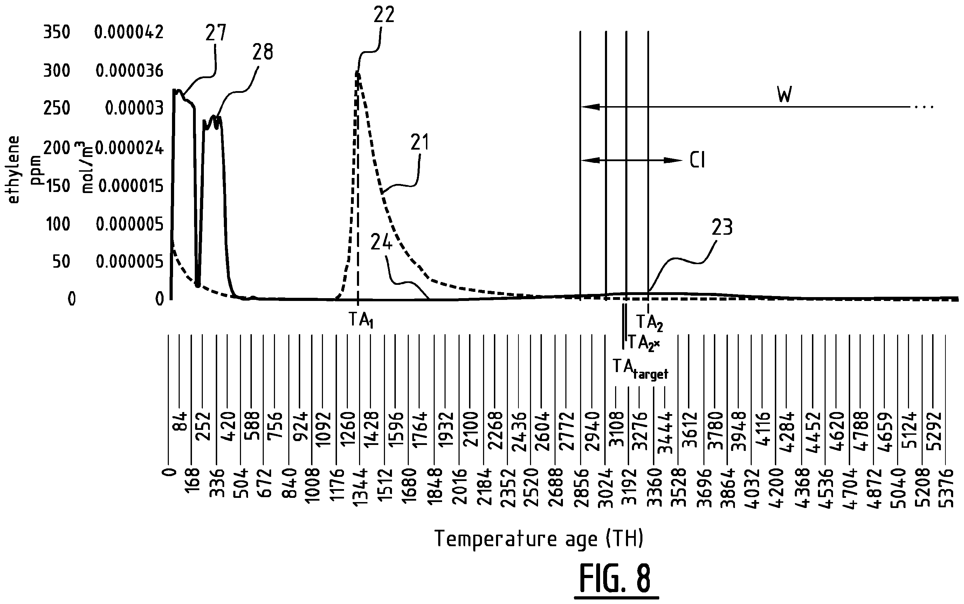

A resulting graph representing ethylene production in fruits as a function of temperature age is shown in Fig. 8. In this graph the calculated ethylene production in the fruits, represented by curve 21, is shown to reach a peak 22 at a first temperature age TA1. In this particular example, TA1 is approximately 1,300 TH. Assuming that the fruits to be ripened are kept at a temperature of 20°C, this would equal a period of 65 hours. Some time will elapse before the ethylene produced in the fruits will have diffused into the surrounding atmosphere. Therefore, a peak in ethylene concentration in the enclosure will be measured only after a delay. This is illustrated in Fig. 8, where the curve 24 representing the measured ethylene production is shown to reach its peak 23 at a second temperature age TA2, which is approximately 3,300 TH. This measured peak in ethylene passed from the fruits to the ambient atmosphere has been found to represent the moment of optimum ripeness of the fruits.

Fig. 8 further shows two early peaks 27, 28 in the measured ethylene curve 24, which are indicative of the introduction of exogenous ethylene into the enclosure. This exogenous ethylene serves to kickstart the ripening process if measured ethylene levels in the enclosure are initially lower than anticipated.

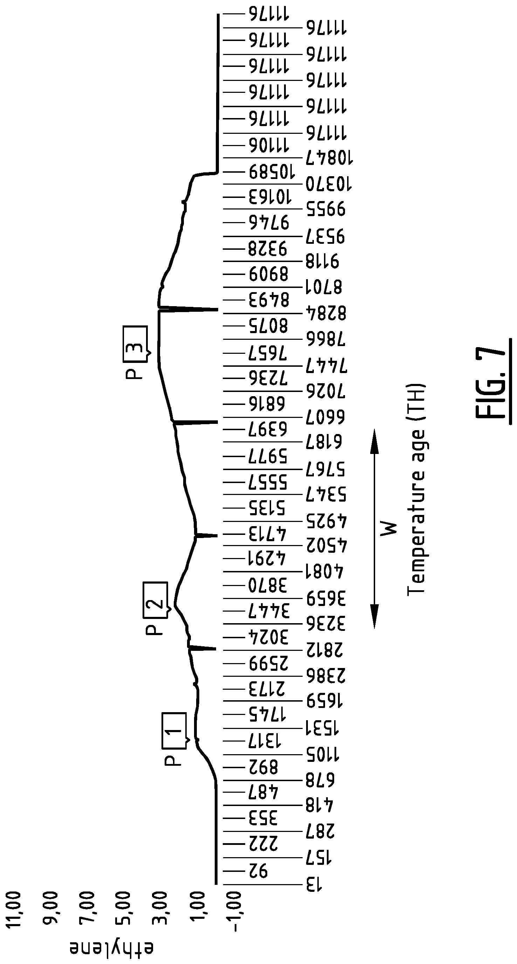

In actual practice, measured values of ethylene in the enclosure or ripening chamber will peak at various points in 'time' (temperature age). This is illustrated in Fig. 7. It has empirically been determined that only one of these peaks is an indication of optimum ripeness of the fruits. The first peak P1 shown in Fig. 7 occurs at a point in time when the fruits are still unripe. The third peak P3, on the other hand, is caused by aging of the fruits after they have reached their optimum ripeness. At that point the fruits are overripe. Only the second peak P2 has been found to relate to the point of optimum ripeness. In the weighted temperature time space defined here, the relevant peak of measured ethylene concentration in the enclosure or ripening chamber should occur within a window or range running from approximately 3,000 TH to approximately 6,500 TH. Again assuming an ambient temperature of approximately 20°C, this would equal a period of 150 to 325 hours, or 6 to 13 days. This range is illustrated by the arrows W in Figs. 7 and 8.

By comparing the first temperature age TA1 where the model calculates the peak value for ethylene production in the fruits with the second temperature age TA2 where the measured ethylene concentration in the enclosure reaches its peak, an average temperature age delay may be determined. Based on this average delay, the value of TA2 for a subsequent ripening process involving another batch of fruits may be predicted once TA1 has been calculated. Because this predicted value TA2* is based on empirical data, it may be expressed as an average or median value, which will be expected to lie within a confidence interval CI. The predicted value TA2* is illustrated in Fig. 8, where the confidence interval CI is shown to overlap with the window W where the peak representing the point of optimum ripeness is expected to occur.

As shown in Fig. 8, the actual measured peak 23 in ethylene is reached at a temperature age TA2 which is very close to the temperature age TA2* as predicted on the basis of the plant physiological model. This means that once the ethylene peak 22 at temperature age TA1 has been identified, the remaining temperature age before reaching the point of optimum ripeness ("ready to eat") at TA2 may be accurately predicted or calculated. Since the conditions in the enclosure (ripening chamber) are known and can be controlled, the actual time remaining can be easily derived from the remaining temperature age and the temperature in the enclosure. This allows a determination to be made when the fruits have to be taken from the enclosure in order to have them at the consumer at the optimum point of ripening. In other words, the fruits may be taken from the enclosure some time before reaching TA2, so that TA2 will be reached at the consumer. In this respect it should be noted that transport from the ripening facility to the consumer will usually take place in refrigerated trucks, so that the duration of this transport does not add any significant temperature age, which further improves the predictability of the degree of ripeness when reaching the consumer.

In accordance with the invention, a target for the degree of ripening in the enclosure will be set such that the fruits will reach the customer in a state which the customer has specified. This customer specified state may e.g. be "ready to eat" or "triggered", i.e. not quite ready to eat. The target degree of ripening will be below the degree of ripening that is achieved when the fruits reach the customer, and the target will thus be met before TA2, e.g. at a temperature age $TA_{target}$ (see Fig. 8). The moment in time when the target will be met may then be predicted or calculated by subtracting the temperature age allowed for transport from the predicted temperature age TA* for optimum ripeness and dividing the result by the known temperature in the enclosure.

The high degree of predictability on the basis of the plant physiological model allows incoming shipments of fruits to be assigned to orders having set delivery dates. Moreover, the model allows the effect of changes in ripening conditions on the moment when the fruits reach their optimum state of ripeness to be calculated. This in turn allows these ripening conditions to be adjusted in order to reach the required state of ripeness at a given moment in time. Since the model is based mainly on two variables, the amount of ethylene and the temperature, making adjustments and measuring their effects can be done relatively swiftly and easily.

In an embodiment of the invention, an alternative plant physiological model is used to monitor the progress of the ripening process. This alternative model is based on the firmness of the fruits as a function of the ethylene concentration in the ambient atmosphere. This model is based on the assumption that softening or loss of firmness F (in N) when the fruits ripen is driven by an active softening enzyme system:

$$\frac{dF}{dt} = -k_F[\mathrm{E}_{soft}](F - F_{fix}), \text{ with } F(t = 0) = F_0 \tag{11}$$

where $k_F$ ($d^{-1}$) is the rate constant for softening, $F_{fix}$ (N) is a fixed component of firmness not affected by wall breakdown, and $F_0$ is the initial firmness. The rate of accumulation of the active softening enzyme system was modelled as the balance of ethylene regulated synthesis from an inactive enzyme precursor, $\mathrm{E}_{soft,\mathrm{pre}}$, and protein turnover:

$$\frac{d[\mathrm{E}_{soft}]}{dt} = k_{\mathrm{E}_{soft}}[\mathrm{E}_{soft,\mathrm{pre}}]\frac{[\mathrm{C_2H_4}]}{\mathrm{K_{m,E}}_{soft} + [\mathrm{C_2H_4}]} - k_{\mathrm{E}_{soft},\deg}[\mathrm{E}_{soft}] \tag{12}$$
$$\text{with} [\mathrm{E}_{soft}](t = 0) = [\mathrm{E}_{soft}]_0$$

$$\frac{d[\mathrm{E}_{soft,\mathrm{pre}}]}{dt} = -k_{\mathrm{E}_{soft}}[\mathrm{E}_{soft,\mathrm{pre}}]\frac{[\mathrm{C_2H_4}]}{\mathrm{K_{m,E}}_{soft} + [\mathrm{C_2H_4}]} \tag{13}$$
$$\text{with}[\mathrm{E}_{soft,\mathrm{pre}}](t = 0) = [\mathrm{E}_{soft,\mathrm{pre}}]_0$$

where $k_{\mathrm{E}soft}$ ($d^{-1}$) and $k_{\mathrm{E}soft,\deg}$ ($d-1$) are the rate constants of the biosynthesis and degradation of the softening enzyme system, respectively; $[\mathrm{E}_{soft}]_0$ and $[\mathrm{E}_{soft,\mathrm{pre}}]_0$ are the initial concentration of the softening enzyme system and its precursor, respectively; $\mathrm{K_{m,E}}_{soft}$ (mol $m^{-3}$) is the Michaelis-Menten constant for regulation of the biosynthesis of the softening enzyme system by ethylene.

Effect of temperature was incorporated into the model by expressing the rate constants as a function of temperature using the Arrhenius equation:

$$k_i = k_{\mathrm{ref,i}}\exp\left(-\frac{Ea_i}{\mathrm{R}}\left(\frac{1}{T} - \frac{1}{T_{\mathrm{ref}}}\right)\right) \tag{14}$$

where $k_i$ and $Ea_i$ (J $mol^{-1}$) are the rate constants and energy of activation, respectively, for reaction i; $k_{\mathrm{ref,i}}$ is the rate constant of the reaction at a reference temperature, $T_{\mathrm{ref}}$ (283 K); R (8.314 J $mol^{-1}$ K) is the universal gas constant, and $T$ (K) is the temperature.

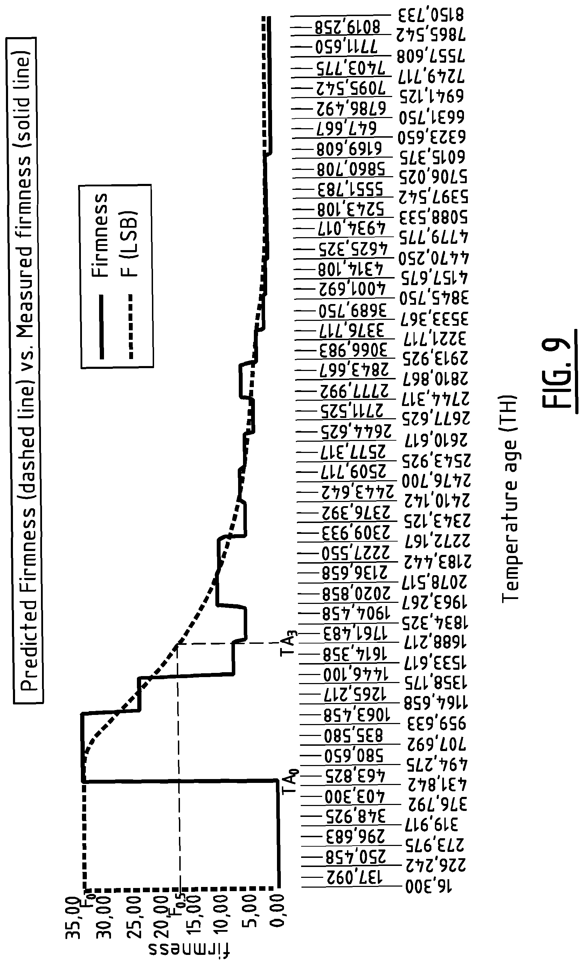

Using this alternative plant physiological model, and using the initial firmness Fo, the temperature $T$ and the ethylene concentration measured in the enclosure as inputs, the firmness at each point in time may be calculated or predicted. The initial firmness $F_0$ may be measured when a batch of fruits is prepared for ripening. Fig. 9 shows a comparison between the predicted firmness based on this alternative model, illustrated by curve 25, and the actual measured firmness of fruits during ripening, illustrated by stepped line 26, both as a function of temperature and time. The scale for temperature and time is again presented in terms of temperature age. The measurements were performed at regular intervals and show a good correlation with the results from the model.

Based on the temperature age and the measured ethylene production, the model determines a momentary value for the firmness. This momentary value provides an indication of the progress of the ripening process. Taking the graph of Fig. 9 as example, the fruits are loaded into the enclosure at TA0, which is approximately 450 TH from the start of a monitoring session. At that time the firmness of the fruits is measured and the measured value is input into the alternative plant physiological model as the initial firmness $F_0$. In this example that value is $F_0$ = 33. From the graph it can be seen that a firmness value of $F_{0.5}$ = 16.5, i.e. half the initial firmness remaining, is reached at a temperature age TA3 of approximately 1,700 TH, or 1,250 TH after ripening in the enclosure started. This value may be signaled as an indication of the progress of the ripening process. For instance, a display on the outside of the enclosure could show a state of ripening of 50%. After the ripening process is ended, based on the targeted degree of ripening being reached, the firmness of the fruits taken from the enclosure or ripening chamber may again be measured. The results of this measurement may be compared to the calculated firmness at that point in time, to confirm that the fruits have indeed reached the targeted degree of ripening. This comparison may further serve to check and calibrate the two plant physiological models.

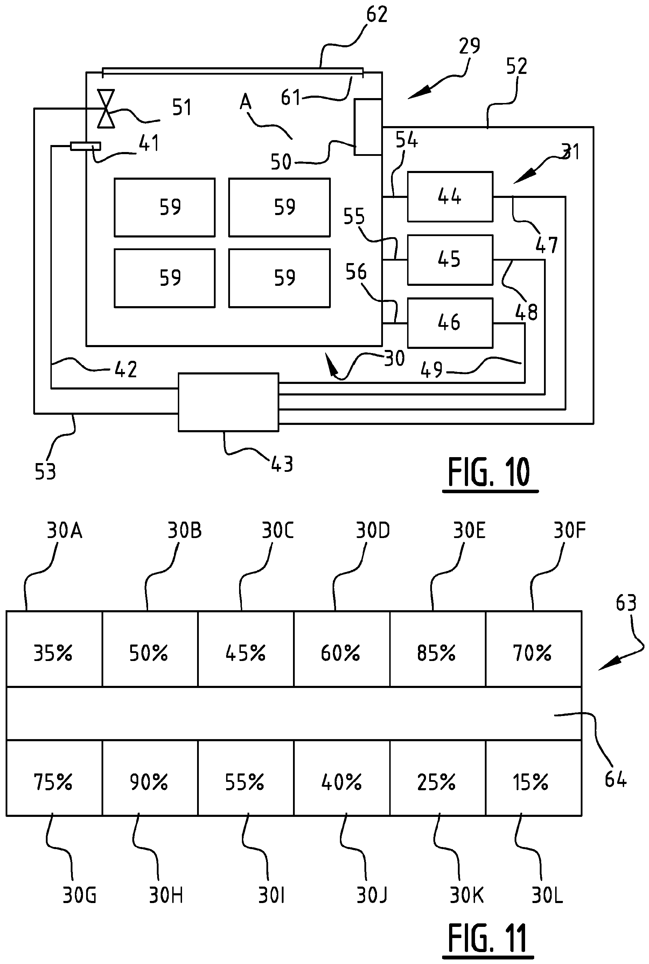

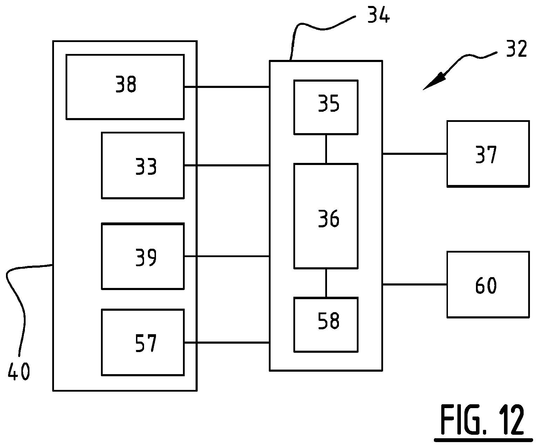

In Fig. 10 a ripening installation 29 is shown. The installation 29 comprises an enclosure 30, an atmospheric control system 31 and a ripening device 32 in accordance with the invention. As shown in Fig. 12, the ripening device 32 comprises a first setting module 33 for setting a target for a degree of ripening of the fruits in the enclosure 30, as well as a control module 34 that is connected to the first setting module 33. The control module 34 includes a plant physiological model 35 describing the ripening process, and a processor 36. The plant physiological model 35 may be stored in a memory. The ripening device 32 further comprises an analyzer module 37 which is connected to the control module 34 and which serves to analyze, at least periodically, a composition of the atmosphere A in the enclosure 30.

The processor 36 is arranged for deriving, based on the model 35, an instantaneous degree of ripening of the fruits from the analyzed composition of the atmosphere A. The processor 36 is further arranged for predicting a moment in time when the target will be met, based again on the model 35 and on the derived degree of ripening. The derivation and prediction may be performed by using the formulae (1) - (8) discussed above. And finally, the processor 36 is arranged for signaling the predicted moment. This may be done e.g. by way of a message shown on a display 38 or by generating some other form of output signal.

In the illustrated embodiment, the ripening device 32 further comprises a second setting module 39 connected to the control module 34 for setting, within a predetermined bandwidth, a moment in time when the target must be met. In this embodiment the processor 36 is further arranged for comparing the set moment with the predicted moment. If the predicted moment differs from the set moment, the processor 36 is further arranged to determining, based on the plant physiological model 35, a value of at least one atmospheric parameter in the enclosure 30 that will allow the predicted moment to match the set moment. And finally, the processor 36 is arranged to control the atmospheric control system 31 to set the determined value of the at least one atmospheric parameter, which may e.g. be the temperature $T$ in the enclosure 30.

The first and second setting modules 33, 39 are shown to form part of a user interface (UI) 40. This user interface 40 is a graphical user interface (GUI), since it also includes the display 38.

In the illustrated embodiment, the analyzer module 37 comprises a sensor 41 which is arranged in the enclosure 30 (Fig. 10). This sensor 41 may act periodically or continuously to generate signals which are representative of the amount of one or more components in the atmosphere A in the enclosure 30. Although only a single sensor 41 is shown, the device may include a plurality of sensors for various components. The sensor signals are sent to a control unit 43 over a line 42. In the illustrated embodiment this control unit 43 includes elements of the ripening device 32 and further includes controls for controlling the atmospheric control system 31.

In this embodiment, the atmospheric control system 31 includes a unit 44 for controlling an amount of oxygen in the enclosure 30, a unit 45 for controlling an amount of carbon dioxide in the enclosure 30, and a unit 46 for controlling an amount of ethylene in the enclosure 30. These various units 44-46 are connected to the control unit 43 by respective lines 47, 48 and 49, and to the enclosure 30 by respective pipes or ducts 54, 55 and 56. The atmospheric control system 31 is further shown to include a unit 50 for controlling a temperature in the enclosure 30, and a unit 51 for circulating the atmosphere A within the enclosure 30. The temperature control unit 50 and air circulation unit 51 are connected to the control unit 43 by respective lines 52 and 53.

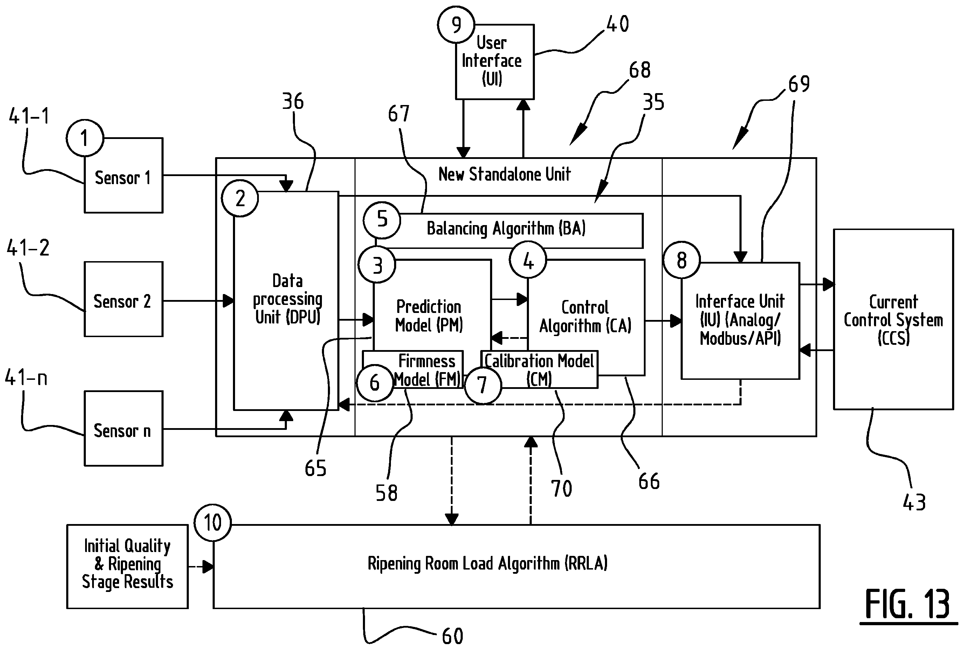

In the illustrated embodiment of the ripening device 32, the plant physiological model 35 includes a prediction algorithm or prediction model 65 (Fig. 13) for iteratively predicting or calculating, based on the analyzed composition of the atmosphere A in the enclosure 30, a future degree of ripening. The model 35 further includes a control algorithm 66 for calculating a setting of the at least one parameter, e.g. the temperature $T$, defining the atmosphere A. The control algorithm 66 may use the output of the prediction algorithm 65 and calculate optimum conditions for the atmosphere A in the enclosure based on the initial setting(s) using the formulae presented above. The prediction algorithm 65 may anticipate changes in the atmosphere A directed by the control algorithm 66.

In this embodiment, the plant physiological model 35 further comprises a balancing algorithm 67 for incrementally adjusting settings of the control algorithm 66 until the future degree of ripening as predicted by the prediction algorithm 65 corresponds to the target. The balancing algorithm 67 allows the prediction and control algorithms 65, 66 to cooperate and use each other's outputs.

This is further illustrated in Fig. 13, where the various elements of the plant physiological model 35 are shown in more detail. In the illustrated embodiment, all the required hardware and software components are shown to be incorporated in a standalone unit (SU) 68 that is physically separated from the control unit 43 for the atmospheric control system 31 (identified here as current control system - CCS). The output signals generated by the SU 68 are communicated to the CCS 43 by means of an interface unit (IU) 69. The interface unit 69 comprises bidirectional hardware that sends outputs of the control algorithm 66 as signals that the CCS 43 can interpret, and that further reads signals from the CCS 43 and transfers these to the processor or data processing unit (DPU) 36 for use in the ripening device 32. Various interface options can be:

- analog setpoints-signals: either voltage or currents will be supplied to the CCS 43, which requires dedicated hardware;
- digital modbus setpoints-signals: the setpoints will be digitally transmitted over a dedicated modbus network, which also requires dedicated hardware;
- Application Programming Interface (API), which requires a software interpreter both at the side of the SU 68 and on the CCS 43.

In this embodiment the processor/DPU 36 receives inputs from a plurality of sensors 41-1 to 41-n, which measure the various components which define the composition of the atmosphere A in the enclosure 30, e.g. oxygen, carbon dioxide and ethylene, as well as the temperature $T$ within the enclosure. The processor/DPU 36 converts the output of the sensors to interpretable values and stores these values in a memory (not shown).

As indicated above, the plant physiological model 35 includes the prediction algorithm or model (PM) 65 that iteratively predicts or calculates the expected moment of ripeness with each measurement, and which anticipates for changed settings of the control algorithm (CA) 66. The control algorithm 66 in turn takes the output from the prediction model 65 in the form of a "Date/Time of Ripeness" and calculates the ideal conditions in the enclosure 30 based on the initial settings. And finally, the balancing algorithm (BA) 67 incrementally changes the settings of the control algorithm 66 until the prediction moment of the prediction algorithm 65 matches the required ripening process finish moment (RPFM).

The SU 68 also comprises a calibration model (CM) 70, which serves to improve the accuracy of the prediction model 65.

The user interface (UI) 40 which communicates with the standalone unit 68 is arranged to send, receive and display i.a. input settings for the various parameters, an expected time-block of ripeness, current and expected average firmness of the batch, status of output signals to the CCS 43, etc.

In the illustrated embodiment, the ripening device 32 further comprises a third setting module 57 connected to the control module 34 for setting an initial value for a degree of ripening of the fruits in the enclosure 30. The third setting module 57 is also shown to form part of the GUI 40. In this case, the control module 34 and the third setting module 57 are configured to determine the initial value based on a measured firmness $F_0$ of the fruits prior to ripening in the enclosure 30. The initial firmness $F_0$ is determined in a non-destructive way by an operator using a suitable instrument, i.e. a firmness detector, and is then entered through the GUI 40. Here, the control module 34 further comprises an alternative plant physiological model 58 describing the ripening process in the fruits. The control module 34 is arranged for deriving a progress of the ripening from the periodically analyzed composition of the atmosphere A in the enclosure 30.

As shown in Fig. 13, the alternative plant physiological model 58, also identified as firmness model (FM) is also comprised in the standalone unit 68. As explained above, this model 58 includes an algorithm that takes the measured components of the atmosphere A, in particular the amount of ethylene, and calculates the corresponding and future or expected average firmness of the batch of fruits. In addition to monitoring the progress of the ripening process, the output of this firmness model 58 can be used by an operator or "Ripening Master" to confirm the validity of the standalone unit 68.

In the illustrated embodiment, multiple batches or pallets 59 of fruits are ripened in the enclosure 30. In order to ensure a mostly uniform ripening process, the ripening device 32 further comprises a module 60 for selecting and positioning the batches or pallets 59 to be placed in the enclosure 30 based on corresponding initial values of their degrees of ripening. In this embodiment, the selection module 60 is configured for reading initial values - for firmness or degree of ripening - for a plurality of batches, comparing these initial values with each other and selecting pairs or groups of pallets 59 of which the initial values of the fruits correspond sufficiently closely. The selection module 60 is further arranged to provide an output - through the GUI 40 - signaling which batches or pallets 59 of fruits have sufficiently closely corresponding initial values of the degree of ripening to be further ripened together in the enclosure 30. This is done through a so-called ripening room load algorithm (RRLA). These pallets 59 identified by the RRLA may then be brought into the enclosure 30, e.g. a ripening chamber, through a doorway 61 which is subsequently hermetically sealed by a door 62. In case of small variations in the degree of ripeness between the various batches or pallets 59, the pallets which carry relatively riper fruits will be positioned closest to the doorway 61. These fruits may be expected to be the first to reach the targeted degree of ripeness, and positioning them in this way allows them to be removed from the ripening chamber 30 without having to handle the pallets 59 carrying fruits which have not yet sufficiently ripened.

As shown in Fig. 13, the selection module 60 including the ripening room load algorithm (RRLA) may be separate from, but in communication with the standalone unit 68.

In Fig. 11 a ripening facility 63 is shown which includes twelve ripening chambers 30A-30L arranged in two rows on opposite sides of a corridor 64. Each of these ripening chambers or enclosures 30A-L contains a substantially uniform load of fruits, which have all been ripened to substantially the same degree. The contents of each enclosure 30A-L may include multiple pallets, which may have been selected and positioned by the selection module 60 discussed above. The instantaneous degree of ripening of the fruits in each enclosure 30A-L may be determined by the second plant physiological model 58 on the basis of the initial firmness $F_0$ and subsequent measurements or calculations of the amount of ethylene in the enclosure 30A-L, as illustrated in Fig. 9. The instantaneous degree of ripening may be displayed on the GUI 40 of the ripening device 32 or on a dedicated display arranged on each enclosure 30A-L. Instead of a numerical representation, the degree of ripening may also be represented visually, e.g. by a running bar or line of lights, or by a gradual change in hue of a signaling light.

In this way the invention provides a method and a device with which the ripening process of climacteric fruits like e.g. avocados or bananas can be easily monitored and a moment of achieving a desired degree of ripeness can be accurately predicted. Moreover, the method and device of the invention allow the ripening process to be controlled by adjusting only a limited number of atmospheric parameters.

Although the invention has been described by reference to various embodiments, it will be apparent that it is not limited thereto, and that many variations of the method and device are conceivable. The scope of the invention is defined solely by the annexed claims.

**Claims**

**1.** A computer implemented method for ripening climacteric fruits in an enclosure comprises the steps of:

    a. setting a target for a degree of ripening of the fruits in the enclosure;
    b. at least periodically analyzing a composition of the atmosphere in the enclosure;
    c. deriving, by a processor, based on a plant physiological model describing the ripening process in the fruits, an instantaneous degree of ripening of the fruits from the analyzed composition;
    d. predicting, by the processor, based on the model and the derived degree of ripening, a moment in time when the target will be met; and
    e. signaling, by the processor, the predicted moment,

wherein the plant physiological model is based on a biosynthetic pathway of ethylene in the fruit and on dilution and diffusion of ethylene through skins of the fruits.

**2.** The computer implemented method of claim 1, wherein the model describes the ripening process based on production of ethylene in the fruits, and the moment in time when the target will be met is predicted after the production of ethylene in the fruits has reached a peak.

**3.** The computer implemented method of claim 1 or 2, wherein the model describes the ripening process as a function of a weighted combination of temperature and time.

**4.** The computer implemented method of claim 2 or 3, wherein the model defines a relationship between the ethylene production in the fruits and the amount of ethylene in the atmosphere in the enclosure; and/or

    wherein the target is associated with a peak in the amount of ethylene in the atmosphere; and optionally
    wherein the model defines the atmospheric peak as point of substantially complete ripening of the fruits when it is reached within a predetermined range of temperature and time.

5. The computer implemented method of any one of the preceding claims, wherein the target to be set is determined based on a desired degree of ripening of the fruits at an consumer and an expected ripening during transport from the enclosure to the consumer; and optionally

    wherein based on the model the processor determines the target to be set on the basis of an expected time of transport from the enclosure to the consumer and an expected temperature during transport; and/or
    wherein the target to be set is determined such that the fruit is substantially ripened when reaching the consumer.

6. The computer implemented method of any one of the preceding claims, further comprising the steps of:

    f. setting, within a predetermined bandwidth, a moment in time when the target must be met;
    g. comparing, by the processor, the set moment with the predicted moment;
    h. if the predicted moment differs from the set moment, determining, by the processor, based on the model, a value of at least one atmospheric parameter in the enclosure that will allow the predicted moment to match the set moment; and
    i. controlling, by the processor, an atmospheric control system connected to the enclosure to set the determined value of the at least one atmospheric parameter.

7. The computer implemented method of any one of the preceding claims, further comprising the steps of:

    j. setting, based on a measured firmness of the fruits prior to ripening in the enclosure, an initial value for a degree of ripening of the fruits; and
    k. deriving, by the processor, based on an alternative plant physiological model describing the ripening process in the fruits, a progress of the ripening from the periodically analyzed composition of the atmosphere in the enclosure; and optionally

wherein multiple batches of fruits are ripened in a single enclosure, and wherein the batches are selected and positioned in the enclosure based on corresponding initial values.

8. A device for ripening climacteric fruits in an enclosure, the device comprising:

    A. a first setting module for setting a target for a degree of ripening of the fruits in the enclosure;
    B. a control module connected to the first setting module and including a plant physiological model describing the ripening process and a processor;
    C. an analyzer module connected to the control module for at least periodically analyzing a composition of the atmosphere in the enclosure;

wherein the processor is arranged for:

    deriving, based on the model, an instantaneous degree of ripening of the fruits from the analyzed composition of the atmosphere;
    predicting, based on the model and the derived degree of ripening, a moment in time when the target will be met; and
    signaling the predicted moment, and

wherein the plant physiological model is based on a biosynthetic pathway of ethylene in the fruit and on dilution and diffusion of ethylene through skins of the fruits.

9. The ripening device of claim 8, wherein the model is configured to describe the ripening process based on production of ethylene in the fruits, and the processor is arranged for predicting the moment in time when the target will be met after the production of ethylene in the fruits has reached a peak.

10. The ripening device of claim 8 or 9, wherein the model is configured to describe the ripening process as a function of a weighted combination of temperature and time.

11. The ripening device of claim 9 or 10, wherein the model is configured to define a relationship between the ethylene production in the fruits and the amount of ethylene in the enclosure; and/or

wherein the target is associated with a peak in the amount of ethylene in the atmosphere; and optionally wherein the model is configured to define the atmospheric peak as point of ripening of the fruits when it is reached within a predetermined range of temperature and time.

12. The ripening device of any one of claims 8-11, wherein the control module and the first setting module are configured to determine the target based on a desired degree of ripening of the fruits at a consumer and an expected ripening during transport from the enclosure to the consumer; and optionally wherein the control module and the first setting module are configured to determine the target based on an expected time of transport from the enclosure to the consumer and an expected temperature during the transport.

13. The ripening device of any one of claims 8-12, further comprising:
D. a second setting module connected to the control module for setting, within a predetermined bandwidth, a moment in time when the target must be met;

wherein the processor is further arranged for:

comparing the set moment with the predicted moment,
if the predicted moment differs from the set moment, determining, based on the model, a value of at least one atmospheric parameter in the enclosure that will allow the predicted moment to match the set moment; and
controlling an atmospheric control system connected to the enclosure to set the determined value of the at least one atmospheric parameter; and optionally

wherein the model includes at least a prediction algorithm for predicting or calculating, based on the analyzed composition of the atmosphere, a future degree of ripening, and a control algorithm for calculating a setting of the at least one parameter defining the atmosphere in the enclosure; and optionally
wherein the model further comprises a balancing algorithm for incrementally adjusting settings of the control algorithm until the future degree of ripening as predicted by the prediction algorithm substantially corresponds to the target.

14. The ripening device of any one of claims 8-13, wherein the first and/or second setting module(s) form(s) part of a user interface; and/or
wherein the analyzer module comprises at least one sensor arranged in the enclosure or connected therewith.

15. The ripening device of any one of claims 13-25, further comprising:
E. a third setting module connected to the control module for setting an initial value for a degree of ripening of the fruits in the enclosure, wherein the control module and the third setting module are configured to determine the initial value based on a measured firmness of the fruits prior to ripening in the enclosure, and
wherein the control module further comprises an alternative plant physiological model describing the ripening process in the fruits, and is arranged for deriving a progress of the ripening from the periodically analyzed composition of the atmosphere in the enclosure; and optionally

wherein the third setting module forms part of a user interface; and/or
wherein the ripening device is configured for ripening multiple batches of fruits in a single enclosure, and further comprises a module for selecting and positioning the batches to be placed in the enclosure based on corresponding initial values.

16. An installation for ripening climacteric fruits, comprising an enclosure, an atmospheric control system connected to the enclosure and the ripening device of any one of claims 8-15 connected to the atmospheric control system.

**FIG. 1**

underripe    Ready to    overripe
             Eat

Temperature age →

**FIG. 2**

R4

R3

R2

R1

Temperature age →

**FIG. 3**

FIG. 4

FIG. 5

FIG. 6

FIG. 7

EP 4 364 575 A1

Temperature age (TH)

## FIG. 8

FIG. 9

FIG. 10

| 30A | 30B | 30C | 30D | 30E | 30F |
|---|---|---|---|---|---|
| 35% | 50% | 45% | 60% | 85% | 70% |

| 75% | 90% | 55% | 40% | 25% | 15% |
|---|---|---|---|---|---|
| 30G | 30H | 30I | 30J | 30K | 30L |

FIG. 11

FIG. 12

FIG. 13

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 3 705 888 A2 (TATA CONSULTANCY SERVICES LTD [IN]) 9 September 2020 (2020-09-09) | 1-16 | INV. A23B7/152 |
| Y | * the whole document * | 1-16 | G01N33/00 G01N33/02 |
| X | WO 2019/096844 A1 (MAERSK CONTAINER IND A/S [DK]) 23 May 2019 (2019-05-23) * the whole document * | 1,5,6,8, 12-14,16 | G06Q10/08 G06Q30/02 G06Q50/02 |
| Y | BRAT P ET AL: "Review of banana green life throughout the food chain: From auto-catalytic induction to the optimisation of shipping and storage conditions", SCIENTIA HORTICULTURAE, ELSEVIER, AMSTERDAM, NL, vol. 262, 29 November 2019 (2019-11-29), XP085964281, ISSN: 0304-4238, DOI: 10.1016/J.SCIENTA.2019.109054 [retrieved on 2019-11-29] * paragraph [2.3.2] * | 3,10 | |
| Y | EP 2 134 877 B1 (EXPRESSIVE RES BV [NL]) 24 April 2013 (2013-04-24) * the whole document * | 7,15 | TECHNICAL FIELDS SEARCHED (IPC) A23B G01N G06Q |

–/––

The present search report has been drawn up for all claims

| Place of search | Date of completion of the search | Examiner |
|---|---|---|
| The Hague | 6 March 2024 | Fiorenza, Francesca |

EPO FORM 1503 03.82 (P04C01)

page 1 of 3

Europäisches
Patentamt

European
Patent Office

Office européen
des brevets

# EUROPEAN SEARCH REPORT

Application Number

EP 23 20 7797

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EBRAHIMI A ET AL: "Novel strategies to control ethylene in fruit and vegetables for extending their shelf life: A review", INTERNATIONAL JOURNAL OF ENVIRONMENTAL SCIENCE AND TECHNOLOGY, CENTER FOR ENVIRONMENT AND ENERGY RESEARCH AND STUDIES (C E E R S), IR, vol. 19, no. 5, 21 June 2021 (2021-06-21), pages 4599-4610, XP037801981, ISSN: 1735-1472, DOI: 10.1007/S13762-021-03485-X [retrieved on 2021-06-21] * page 4600 - page 4601; figure 1 * ----- | 1-16 | |
| Y | Rene Clarisse Tong: "THE EFFECTS OF AMINOETHOXYVINYLGLYCINE (AVG) AND 1-METHYLCYCLOPROPENE (1-MCP) ON BANANA RIPENING", , 1 November 2008 (2008-11-01), XP055112549, Retrieved from the Internet: URL:https://ujdigispace.uj.ac.za/bitstream /handle/10210/3265/Tong.pdf?sequence=1 [retrieved on 2014-04-07] * page 19 - page 22; figure 1.7 * ----- | 1-16 | TECHNICAL FIELDS SEARCHED (IPC) |
| Y | US 2003/150334 A1 (GAEBLER RALPH [US]) 14 August 2003 (2003-08-14) * the whole document * ----- | 1-16 | |

-/--

The present search report has been drawn up for all claims

| Place of search | Date of completion of the search | Examiner |
|---|---|---|
| The Hague | 6 March 2024 | Fiorenza, Francesca |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons
......................................................................................
& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 23 20 7797

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PAUL VIJAY ET AL: "Role of internal atmosphere on fruit ripening and storability-a re", JOURNAL OF FOOD SCIENCE AND TECHNOLOGY, SPRINGER (INDIA) PRIVATE LTD, INDIA, vol. 51, no. 7, 26 November 2011 (2011-11-26), pages 1223-1250, XP035307225, ISSN: 0022-1155, DOI: 10.1007/S13197-011-0583-X [retrieved on 2011-11-26] * the whole document * | 1-16 | |

TECHNICAL FIELDS
SEARCHED     (IPC)

The present search report has been drawn up for all claims

| Place of search | Date of completion of the search | Examiner |
|---|---|---|
| The Hague | 6 March 2024 | Fiorenza, Francesca |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 3 of 3

## ANNEX TO THE EUROPEAN SEARCH REPORT
## ON EUROPEAN PATENT APPLICATION NO.

EP 23 20 7797

This annex lists the patent family members relating to the patent documents cited in the above-mentioned European search report.
The members are as contained in the European Patent Office EDP file on
The European Patent Office is in no way liable for these particulars which are merely given for the purpose of information.

06-03-2024

| Patent document cited in search report | | | Publication date | Patent family member(s) | | | Publication date |
|---|---|---|---|---|---|---|---|
| EP | 3705888 | A2 | 09-09-2020 | EP | 3705888 | A2 | 09-09-2020 |
| | | | | US | 2020281220 | A1 | 10-09-2020 |
| WO | 2019096844 | A1 | 23-05-2019 | CN | 111356371 | A | 30-06-2020 |
| | | | | DK | 201770862 | A1 | 11-06-2019 |
| | | | | EP | 3709813 | A1 | 23-09-2020 |
| | | | | RU | 2020119362 | A | 15-12-2021 |
| | | | | US | 2020275671 | A1 | 03-09-2020 |
| | | | | WO | 2019096844 | A1 | 23-05-2019 |
| EP | 2134877 | B1 | 24-04-2013 | AR | 065409 | A1 | 03-06-2009 |
| | | | | AU | 2008217791 | A1 | 28-08-2008 |
| | | | | BR | PI0807589 | A2 | 01-07-2014 |
| | | | | CA | 2678804 | A1 | 28-08-2008 |
| | | | | CN | 101680028 | A | 24-03-2010 |
| | | | | EP | 2134877 | A1 | 23-12-2009 |
| | | | | ES | 2420836 | T3 | 27-08-2013 |
| | | | | NL | 1033431 | C2 | 21-08-2008 |
| | | | | NZ | 579197 | A | 26-10-2012 |
| | | | | PL | 2134877 | T3 | 30-09-2013 |
| | | | | RU | 2009134951 | A | 27-03-2011 |
| | | | | US | 2010273155 | A1 | 28-10-2010 |
| | | | | WO | 2008103040 | A1 | 28-08-2008 |
| | | | | ZA | 200905929 | B | 26-05-2010 |
| US | 2003150334 | A1 | 14-08-2003 | AT | E306193 | T1 | 15-10-2005 |
| | | | | AU | 5819001 | A | 03-10-2001 |
| | | | | DE | 10013501 | A1 | 29-11-2001 |
| | | | | EP | 1265489 | A2 | 18-12-2002 |
| | | | | US | 2003150334 | A1 | 14-08-2003 |
| | | | | WO | 0171258 | A2 | 27-09-2001 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3705888 A2